# EUROPÄISCHE PATENTANMELDUNG

(11) **EP 2 446 918 A1**
(43) Veröffentlichungstag der Anmeldung: **02.05.2012**
(21) Anmeldenummer: 11184596.2
(22) Anmeldetag: 11.10.2011
(51) Int. Cl.: A61M 25/10, A61B 19/00, A61M 29/02, A61M 25/01

(54) **Ballonkatheter mit einem radial asymmetrisch dilatierbaren Abschnitt**

(30) Priorität: 29.10.2010 US 407921 P
(71) Anmelder: Biotronik AG, 8180 Bülach (CH)
(72) Erfinder: Ulmer, Jens, 8008 Zürich (CH)
(74) Vertreter: Lindner-Vogt, Karin L.

(57) **Zusammenfassung**

Die vorliegende Anmeldung betrifft einen Ballonkatheter mit einem dilatierbaren Ballon, dadurch gekennzeichnet, dass der dilatierbare Ballon mindestens einen radial asymmetrisch dilatierbaren Abschnitt umfasst, wobei der Querschnitt des radial asymmetrisch dilatierbaren Abschnitts mindestens einen ersten Bereich (6) und einen, dem ersten Bereich gegenüberliegenden, zweiten Bereich (5) aufweist und sich der erste und der zweite Bereich in ihrer Radialfestigkeit der Ballonwand voneinander unterscheiden.

## Beschreibung

Die Angioplastie, auch perkutane transluminale Angioplastie (PTA) oder perkutane transluminale Coronarangioplastie (PTCA), ist ein Verfahren zur Erweiterung oder Wiederer-öffnung von verengten oder verschlossenen Blutgefäßen (meistens Arterien, seltener auch Venen). Ein gebräuchliches Verfahren der Angioplastie ist die Ballondilatation.

Unter der Ballondilatation im Rahmen einer Angioplastie versteht man in der interventionellen Radiologie, der Kardiologie und der Angiologie eine Methode zur Aufdehnung krankhaft verengter Blutgefäße mittels eines Ballonkatheters, einem Gefäßkatheter mit daran angebrachtem Ballon, der sich erst an der verengten Stelle langsam unter hohem Druck (6-20 bar) entfaltet. Dadurch werden die Engstellen, die v. a. durch atherosklerotische Veränderungen (Gefäßverkalkung) entstehen, so gedehnt, dass sie den Blutstrom nicht mehr oder weniger stark behindern.

Dabei werden die Ballonkatheter fast immer von der Leiste aus über einen Führungsdraht und Führungskatheter in die Stenose (Engstelle) platziert und mit Druck aufgeblasen. Hierdurch wird meist die Engstelle beseitigt und eine Operation vermieden.

Moderne Methoden im Bereich Kunststoffverarbeitung ermöglichen die Konstruktion und Weiterentwicklung solcher Ballons, um die Qualität individuell auf die Bedürfnisse der Patienten anzupassen. Wichtig sind hierbei die Flexibilität der Ballons sowie ihre Druckfestigkeit.

Die Ballonkatheter des Standes der Technik zeichnen sich dadurch aus, dass sie während der Ballondilatation eine achsensymmetrische Radialkraftverteilung aufweisen. Dadurch ist die radiale Kraft, die auf die Gefäßwände ausgeübt wird, entlang der Längsachse des Ballons im Wesentlichen gleich verteilt. Die meisten zu behandelnden Stenosen weisen allerdings selbst keine radialsymmetrische Ausdehnung innerhalb des Gefäßes auf (sog. exzentrische Stenose). Dies hat zur Folge, dass bei Behandlung solcher exzentrischen Stenosen mittels handelsüblicher Ballonkatheter umliegendes gesundes Gewebe unnötig belastet wird und es dort während der Behandlung zu Verletzungen der Gefäßwand kommen kann. Solche traumatologischen Gefäßwandveränderungen, auch und gerade in gesundem Gewebe, werden heutzutage als wesentlicher Auslöser einer Gewebeneubildung (neointimale Hyperplasie) und damit verbunden mit der Gefahr einer Restenosebildung betrachtet.

Aufgabe der vorliegenden Erfindung ist es, einen oder mehrere Nachteile des Standes der Technik zu vermindern oder zu vermeiden. Insbesondere ist es eine Aufgabe der vorliegenden Erfindung Mittel bereitzustellen, die die Behandlung von Stenosen erlauben, aber umliegendes gesundes Gewebe weniger stark belasten.

Die vorliegende Erfindung löst diese Aufgabe durch Bereitstellung eines Ballonkatheters mit einem dilatierbaren Ballon, dadurch gekennzeichnet, dass der dilatierbare Ballon mindestens einen radial asymmetrisch dilatierbaren Abschnitt umfasst, wobei der Querschnitt des radial asymmetrisch dilatierbaren Abschnitts mindestens einen ersten Bereich und einen, dem ersten Bereich gegenüberliegenden, zweiten Bereich aufweist und sich der erste und der zweite Bereich in ihrer Radialfestigkeit der Ballonwandung (Compliance) voneinander unterscheiden. Bevorzugt wird eine niedrigere Radialfestigkeit durch Wahl einer geringeren Wandstärke erreicht.

Der erfindungsgemäße Ballonkatheter zeichnet sich dadurch aus, dass der effektiv auf die Gefäßwand ausgeübte Druck während der Dilatation asymmetrisch verteilt ist. Bereiche mit einer geringeren Wandstärke erzeugen während der Ballondilatation höhere Scherkräfte, als Bereiche mit einer größeren Wandstärke. Dadurch wird erreicht, dass der effektiv auf die Gefäßwand übertragene resultierende Druck in Bereichen mit geringerer Wandstärke größer ist, als in Bereichen mit relativ dazu größeren Wandstärken. Im Weiteren wird die effektiv auf die Gefäßwand übertragene Scherkraft als resultierender Druck bezeichnet. Der erfindungsgemäße Ballonkatheter erlaubt es nun in besonders vorteilhafter Weise Gefäßengstellen zu behandeln, wobei bevorzugt auf die erkrankten Bereiche der Gefäßwand, welche sich durch eine erhöhte Festigkeit auszeichnen, ein größerer resultierender Druck ausgeübt wird, während der resultierende Druck, der auf die umliegenden gesunden Teile der Gefäßwand ausgeübt wird, aufgrund der erhöhten Festigkeit der Ballonwand in diesem Abschnitt und dadurch bedingt, geringere Scherkräfte verursacht, geringer ist. So kann eine Stenose effektiv beseitigt werden, während das umliegende gesunde Gefäßgewebe nur wenig belastet wird. Durch Verwendung des erfindungsgemäßen Ballonkatheters wird ohne Einsatz von Medikamenten oder Wirkstoffen die Gewebeneubildung (neointimale Hyperplasie) verringert.

Grundsätzlich kann für den erfindungsgemäßen Ballonkatheter jedes bekannte Ballonkathetersystem mit einem dilatierbaren Ballon verwendet werden. Insbesondere betrifft die Erfindung einen Ballonkatheter mit einem Innenschaft, an dem am distalen Ende ein dilatierbarer Ballon befestigt ist, der in einem nicht-expandierten, deflatierten Zustand an einer Außenoberfläche des Innenschaftes zumindest teilweise anliegt. Die Erfindung betrifft insbesondere solche Katheter, die einen dilatierbaren Ballon aufweisen, dessen Außenoberfläche nach dem Einführen des Katheters in ein Gefäß und anschließender Dilatation des Ballons am gewünschten Gefäßabschnitt mindestens in Teilen an eine Gefäßwand gedrückt wird.

Ballonkatheter der vorgesehenen Art weisen üblicherweise neben einem Innenschaft und dem dilatierbaren Ballon auch einen Außenschaft auf, der wenigstens bis zu einem proximalen Ende des Ballons reicht und mit diesem fluiddicht verbunden ist. Zwischen Innen- und Außenschaft des Katheters ist üblicherweise eine in Längsrichtung des Katheters von seinem proximalen Ende bis ins Innere des Ballons reichende Fluidleitung vorgesehen, die sich beispielsweise daraus ergibt, dass der Außenschaft einen Innendurchmesser besitzt, der größer ist, als ein Außendurchmesser des Innenschaftes.

Im Inneren des Innenschaftes ist ein vom Innenschaft eingeschlossener, sich in Längsrichtung des Innenschaftes erstreckender Hohlraum als Lumen vorgesehen. Dieses Lumen dient beispielsweise der Aufnahme eines Mandrins oder eines Führungsdrahtes. Katheter und Führungsdraht sind dann beispielsweise so ausgebildet, dass der Führungsdraht an der distalen Spitze des Katheters austreten kann und vom proximalen Ende her zu steuern ist. Der Führungsdraht wird zum Beispiel mit Hilfe von Steuermitteln so ausgelenkt, dass er auch in abzweigende Blutgefäße leicht einzuführen ist. Der Ballonkatheter kann dann entlang des Führungsdrahtes nachgeschoben werden.

Unabhängig von der Art des Katheters, insbesondere hinsichtlich der Gestaltung der Führungsmittel, weisen Ballonkatheter an ihrem distalen Ende den bereits erwähnten, dilatierbaren Ballon auf. Während des Einführens des Ballonkatheters ist der Ballon komprimiert und liegt eng am Innenschaft des Katheters an. Durch Inflatieren des Ballons mit einem Fluid kann dieser expandiert werden. Dieses Expandieren oder Dilatieren des Ballons geschieht, sobald der Ballon bis zu der bestimmungsgemäßen Position geführt ist. Durch das Expandieren des Ballons wird eine Oberfläche des Ballons an eine Gefäßwand anlegt. Dies geschieht beispielsweise zu dem Zweck, Gefäßverengungen (Stenosen) mittels des Ballonkatheters zu weiten.

Der erfindungsgemäße Ballonkatheter kann mindestens auf Teilen der Außenoberfläche des dilatierbaren Ballons, bevorzugt ausschließlich auf Teilen oder der gesamten Außenoberfläche des oder der radial asymmetrisch dilatierbaren Abschnitte des Ballonkatheters, eine wirkstoff-freisetzende Beschichtung und/oder Kavitätenfüllung aufweisen. Verfahren zur Beschichtung von Ballonkathetern und zur Anbringung von Kavitätenfüllungen auf Ballonkathetern sind dem Fachmann bekannt.

Der dilatierbare Ballon des erfindungsgemäßen Ballonkatheters weist mindestens einen asymmetrisch dilatierbaren Abschnitt auf. Unter einem Abschnitt wird dabei ein radial die Längsachse umgebender Teil des dilatierbaren Ballons verstanden mit einer Ausdehnung in längsaxialer Richtung. Ein Abschnitt kann beispielsweise durch zwei parallele, im Winkel von 90° zur Längsachse des dilatierbaren Ballons verlaufende Schnittebenen (Querschnitte) begrenzt werden, die in Richtung der Längsachse des Ballons um eine bestimmte Distanz versetzt sind. Ein Querschnitt durch einen Abschnitt stellt somit gleichzeitig auch einen Querschnitt durch den dilatierbaren Ballon des Ballonkatheters dar.

Der radial asymmetrisch dilatierbare Abschnitt des erfindungsgemäßen Ballonkatheters weist einen Querschnitt auf, der mindestens einen ersten und einen, dem ersten Bereich gegenüberliegenden, zweiten Bereich umfasst. Dabei liegen sich die beiden Bereiche dann gegenüber, wenn sich die Mittelpunkte der Bereiche punktsymmetrisch durch den Mittelpunkt des Querschnitts auf dem Umfang des Querschnitts gegenüberliegen. Erster und zweiter Bereich bilden dabei Teile des Umfangs des Querschnitts durch den Abschnitt. Der Querschnitt durch den Abschnitt ist dabei bevorzugt so gelegt, dass der Querschnittsumfang die maximale radiale Ausdehnung des zweiten Bereichs beinhaltet. Im einfachsten Fall wird der gesamte Umfang des Querschnitts des Abschnitts aus einem ersten und zweiten Bereich gebildet. Der Umfang des Querschnitts des Abschnitts kann aber auch noch weitere, andere Bereiche aufweisen. Es ist auch möglich, dass der Querschnitt eines radial asymmetrisch dilatierbaren Abschnitts mehr als einen ersten und zweiten Bereich aufweist. Ein zweiter Bereich bildet bevorzugt mindestens 0,01% und nicht mehr als 50% des Querschnittsumfangs.

Der erste und zweite Bereich des Querschnitts des radial asymmetrisch dilatierbaren Abschnitts unterscheiden sich mindestens in der Wandstärke des jeweiligen Bereiches. Bevorzugt ist die Wandstärke des zweiten Bereichs geringer als die Wandstärke des ersten Bereichs. Dieser Unterschied in der Wandstärke führt dazu, dass der zweite Bereich während des Dilatierens einen höheren resultierenden Druck auf eine umliegende Gefäßwand überträgt als der erste Bereich des Ballonkatheters. Bevorzugt ist an der, der minimalen Wandstärke des zweiten Bereichs gegenüberliegenden, Stelle des Querschnittsumfangs die Wandstärke des ersten Bereichs größer als die Wandstärke an der Stelle des zweiten Bereichs mit minimaler Wandstärke. Dadurch soll gewährleistet werden, das sich die Kraftverteilung, hervorgerufen durch die unterschiedlichen Scherkräfte, während der Dilatation an die Festigkeitsunterschiede der umliegenden Gefäßwand in der Weise anpasst, dass überwiegend die erkrankten Gefäßbereiche aufgedehnt werden und die gesunden Gefäßbereiche nur wenig belastet werden. Dazu ist es vorteilhaft, die Ballonwandstärke des zweiten Bereichs so zu wählen, das der resultierende Druck im zweiten Bereich mindestens gleich, im besten Fall aber geringer ist als der resultierende Druck im ersten Bereich. Somit wirkt eine höhere Kraft auf die erkrankten Gefäßabschnitte und verhindert so ein zu starkes überdehnen der gesunden Gefäßabschnitte.

Der erfindungsgemäße Ballonkatheter kann sich insbesondere dadurch auszeichnen, dass das Verhältnis δ der minimalen Wandstärke des zweiten Bereichs zur Wandstärke der der minimalen Wandstärke des zweiten Bereichs gegenüberliegenden Stelle des ersten Bereichs 1>δ≥0,01 beträgt, bevorzugt 0,95≥δ≥0,1, besonders bevorzugt 0,9≥δ≥0,7.

Dabei ist δ = p/q; mit
p = Wandstärke an der Stelle mit minimaler Wandstärke des zweiten Bereichs auf dem Querschnittsumfang; und
q = Wandstärke des ersten Bereichs an der Stelle, die der minimalen Wandstärke des zweiten Bereichs auf dem Querschnittsumfang gegenüberliegt.

Das Verhältnis δ beträgt dabei 1>δ≥0,01; bevorzugt 0,95≥δ≥0,1.

Dieses Prinzip funktioniert insbesondere dann, wenn der Ballon die Stenose im fast vollständig dilatierten Zustand öffnen soll. D.h. zu Begin der Inflation wird das gesamte Gewebe zunächst elastisch gedehnt. Erst ab einem bestimmten Druck und einer fast vollständigen Ausdehnung des Ballonkatheters wirkt sich die unterschiedliche Dehnfähigkeit der verschieden starken Ballonwandungen aus, um das umliegende Gewebe auch plastisch und dauerhaft durch die auftretenden Scherkräfte zu verformen. Diese plastische Verformung erfolgt bei Ballonkathetern nach dem Stand der Technik ausschließlich in Gewebeabschnitten mit der geringsten Festigkeit. Da dies meist die gesunden Bereiche sind, soll mit der hier vorliegenden Erfindung genau dieses vermieden werden, indem die gesunden Gefäßabschnitte durch eine erhöhte Festigkeit der Ballonwand geschützt werden

Der erfindungsgemäße Ballonkatheter kann auch bei der Behandlung von sogenannten vulnerablen Plaques von Vorteil sein. Hierbei handelt es sich um erkrankte Gefäßbereiche, bei denen eine unstabile zum Einreisen neigende Einkapselung in der Gefäßwand enthalten ist. Vulnerable Plaques zeichnen sich durch eine erhöhte Thrombosegefahr nach Plaqueruptur aus. Somit ist es wünschenswert diese erkrankten Bereiche besonders schonend zu behandeln, was durch den hier beschriebenen Ballonkatheter ermöglicht wird.

Die Form, Positionierung und Anzahl von radial asymmetrisch dilatierbaren Abschnitten des erfindungsgemäßen Ballonkatheters bzw. der zweiten Bereiche dieser Abschnitte richtet sich im Wesentlichen nach der Form, der Lage und der Ausdehnung des zu behandelnden Areals im Gefäß.

Der erfindungsgemäße Ballonkatheter kann beispielsweise lediglich einen einzigen radial asymmetrisch dilatierbaren Abschnitt aufweisen.

Der erfindungsgemäße Ballonkatheter kann auch mehrere getrennt voneinander vorliegende, aufeinander folgende oder überlappende radial asymmetrisch dilatierbare Abschnitte aufweisen.

Es ist auch möglich, dass einer oder mehrere der radial asymmetrisch dilatierbaren Abschnitte des erfindungsgemäßen Ballonkatheters mehr als einen zweiten Bereich aufweisen.

Die radial asymmetrisch dilatierbaren Abschnitte können einen oder mehrere zweite Bereiche aufweisen, die in einer bestimmten geometrischen Form gestaltet sind. Die zweiten Bereiche können beispielsweise unabhängig voneinander kreisförmig, elliptisch oder streifenförmig ausgebildet sein.

Der erfindungsgemäße Ballonkatheter kann sich beispielsweise dadurch auszeichnen, dass er einen einzigen radial asymmetrisch dilatierbaren Abschnitt aufweist und dieser sich im Wesentlichen über den gesamten dilatierbaren Ballon erstreckt.

Weist der erfindungsgemäße Ballonkatheter mehr als einen radial asymmetrisch dilatierbaren Abschnitt auf, so kann diese Mehrzahl derart ausgeführt und angeordnet sein, dass die Summe der Längsausdehnungen der einzelnen radial asymmetrisch dilatierbaren Abschnitte nicht mehr als 50% der Längsausdehnung des dilatierbaren Bereichs des Ballonkatheters beträgt.

Das Vorhandensein mehrerer bevorzugt dilatierbarer zweiter Bereiche auf einem erfindungsgemäßen Ballonkatheter, sei es im Rahmen eines einzigen oder mehrerer radial asymmetrisch dilatierbarer Abschnitte, kann bei geeigneter Ausgestaltung und Positionierung der zweiten Bereiche beispielsweise auch dazu dienen eine unerwünschte Lageänderung des Ballons während der Dilatation zu verhindern. Insbesondere können die zum therapeutisch eingesetzten zweiten Bereich zusätzlich vorhandenen Bereiche derart ausgerichtet und ausgestaltet sein, dass eine ungewollte Rotationsbewegung des Ballons während des Dilatierens im Gefäß vermieden oder mindestens erschwert wird. Zu einer solchen Lageänderung kann es beispielsweise dann kommen, wenn der bevorzugt gedehnte zweite Bereich auf die zu dehnende Läsion trifft und von dieser abgelenkt wird oder von ihr abrutscht bevor der Rest des Ballons ausreichend gedehnt wurde um eine stabile Lage im Gefäß sicherzustellen und damit eine ungewollte Rotationsbewegung im Gefäß zu unterbinden.

Um die Vorteile des erfindungsgemäßen Ballonkatheters besonders effektiv nutzen zu können, ist es sinnvoll, dafür zu sorgen, dass der Ballonkatheter vor der Dilatation am gewünschten Ort im Gefäß derart radial ausgerichtet ist, dass der bevorzugt dilatierbare zweite Bereich des radial asymmetrisch dilatierbaren Abschnitts auch auf der zu behandelnden Stelle zu liegen kommt und nicht auf umliegendem gesundem Gewebe.

Für den Erfolg der asymmetrischen Kraftverteilung durch den erfindungsgemäßen Ballonkatheter ist es also wünschenswert, dass der Ballonkatheter innerhalb des Gefäßes exakt positionierbar ist. Dabei muss nicht nur die longitudinale Position gewährleistet werden, sondern auch der Ballon durch Rotation in die richtige radiale Ausrichtung gebracht werden. Dem Fachmann ist dabei bekannt, das außer der unten beschriebenen Methode, weitere Methoden und bildgebende Verfahren existieren, um die exakte Positionierung eines Ballonkatheters innerhalb des Gefäßes zu erzielen. So kann z.B. mit Hilfe von intravaskulären Bildgebungsverfahren (Ultraschall, optische Kohäreztomographie) in Kombination mit einem Ballonkatheter, dieser exakt innerhalb des Gefäßes ausgerichtet werden. Weiterhin gibt es Verfahren, die die räumliche Lage eines Katheters innerhalb eines Lumens unter Zuhilfenahme von Permanentmagneten in der Katheterspitze bestimmen.

Für ein einfacheres Verfahren kann auch folgendes Prinzip angewendet werden:
Die Oberfläche des Innenschafts und/oder Außenschafts kann einen oder mehrere Marker aufweisen, die derart angeordnet und orientiert sind, dass während der Applikation des Ballonkatheters im Gefäß des zu behandelnden Individuums die räumliche Ausrichtung des radial asymmetrisch dilatierbaren Abschnitts und damit insbesondere die Lage des bevorzugt dilatierbaren zweiten Bereichs bestimmbar ist. Bevorzugt sind die Marker derart positioniert und gruppiert, dass die Rotationsstellung des radial asymmetrisch dilatierbaren Abschnitts im Gefäß bestimmbar ist.

Dabei werden bevorzugt Marker eingesetzt, die sich von außerhalb des Körpers mittels bildgebender Verfahren erfassen und darstellen lassen. Dem Fachmann sind geeignete Marker bekannt. Beispielsweise kann es sich um bekannte Röntgen- und/oder Fluoreszenzmarker handeln. Um eine sichere Detektion und Darstellung der dreidimensionalen Lage des Ballonkatheters im Gefäß zu erlauben, werden bevorzugt mehr als eine Markersubstanz bzw. mehr als ein Marker eingesetzt, besonders bevorzugt mindestens zwei verschiedene Marker, ganz besonders bevorzugt mindestens drei verschiedene Marker. Durch den Einsatz mehrerer verschiedener Marker wird das Risiko von Artefakten in der Bildgebung verringert.

Dabei werden die Marker bevorzugt in unterschiedlichen geometrischen Figuren auf der Oberfläche des Innenschafts und/oder Außenschafts des Katheters angeordnet. Bei den geometrischen Figuren handelt es sich bevorzugt um Ringe, Bänder, Dreiecke und/oder Pfeile, wobei die erstgenannten als Referenzsysteme zur späteren Bildverarbeitung dienen. Diese geometrischen Figuren können dabei derart gestaltet werden, dass trotz einer zweidimensionalen Bildgebung (in einer Art "Draufsicht") die Orientierung des Ballonkatheters im dreidimensionalen Raum bestimmbar ist. Dazu können verschiedene Figuren miteinander kombiniert werden. Besonders vorteilhaft ist die Verwendung von Dreiecken, die sich insbesondere über den halben Katheterumfang erstrecken können. In einer bevorzugten Ausführungsform werden mindestens drei gleiche oder verschiedene geometrische Figuren derart auf der Oberfläche des dilatierbaren Ballons angebracht und ausgerichtet, so dass die Rotationsstellung des oder der radial asymmetrisch dilatierbaren Abschnitte bestimmbar ist.

Bevorzugt werden mindestens zwei Dreiecke eingesetzt, deren Spitzen gegenläufig angeordnet sind. Besonders bevorzugt werden drei Dreiecke verwendet, deren Spitzen alternierend gegenläufig zueinander angeordnet sind und deren Basis jeweils derart zueinander angeordnet ist, dass ein für jede Rotationslage des Ballonkatheters spezifisches, eindeutiges zweidimensionales Bild erhalten wird. Dieses Bild kann mit Hilfe von Bildanalyseverfahren automatisch analysiert werden und direkt in eine Rotationsposition umgerechnet werden. Dabei dienen die Radiomarker an den Enden des Ballons als Referenzsysteme, die es erlauben, sowohl die Fläche als auch die geometrische Form (in ihrer zweidimensionaler Darstellung) der dreieckigen Positionsmarkierungen eindeutig zu bestimmen.

Figuren:

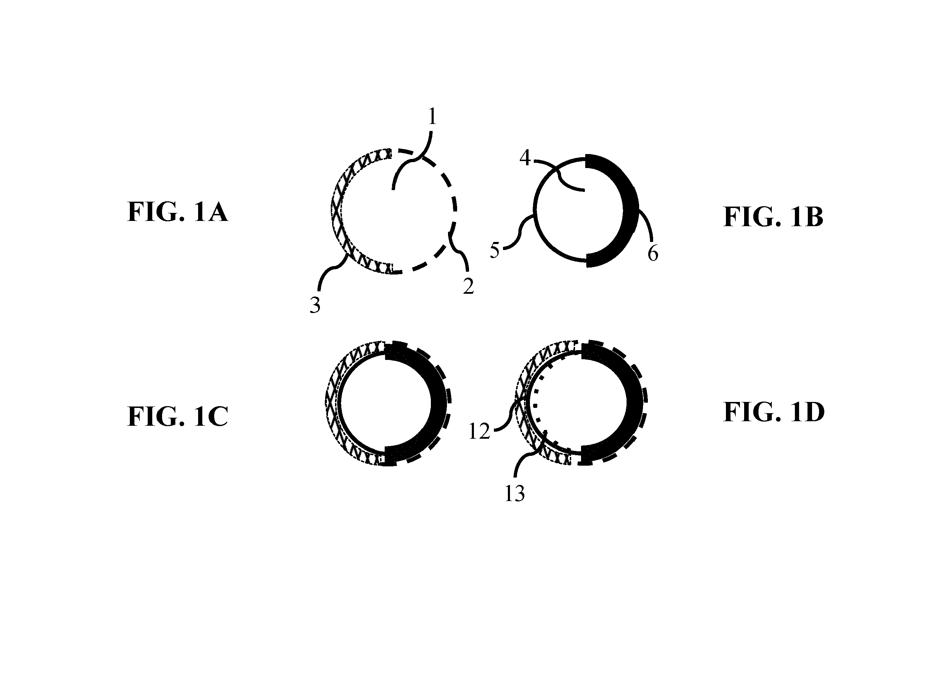

- Figur 1: zeigt eine schematische Darstellung einer ersten Ausführungsform des erfin-dungsgemäßen Ballonkatheters sowie dessen Verwendung. (A) schematische Darstellung eines Querschnitts durch ein zu behandelndes Gefäß; (B) schema-tische Darstellung eines Querschnitts durch einen radial asymmetrisch dilatier-baren Abschnitt des Ballonkatheters; (C) Positionierung des erfindungsgemä-ßen Ballonkatheters im zu behandelnden Gefäß; (D) Zustand nach erfolgter Po-sitionierung und Dilatation des Ballons.

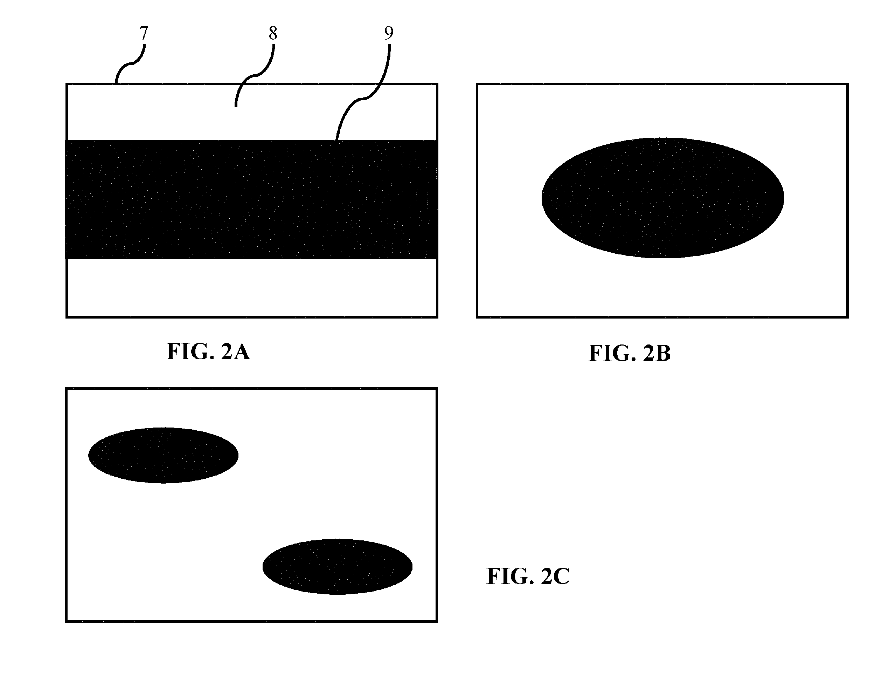

- Figur 2: zeigt eine schematische Darstellung der Gestaltung von radial asymmetrisch dilatierbaren Abschnitten eines erfindungsgemäßen Ballonkatheters. (A) Dar-stellung eines radial asymmetrisch dilatierbaren Abschnitts mit streifenförmi-gem zweitem Bereich; (B) Darstellung eines radial asymmetrisch dilatierbaren Abschnitts mit ellipsenförmigem zweitem Bereich; (C) Darstellung eines radial asymmetrisch dilatierbaren Abschnitts mit zwei ellipsenförmigen zweiten Be-reichen.

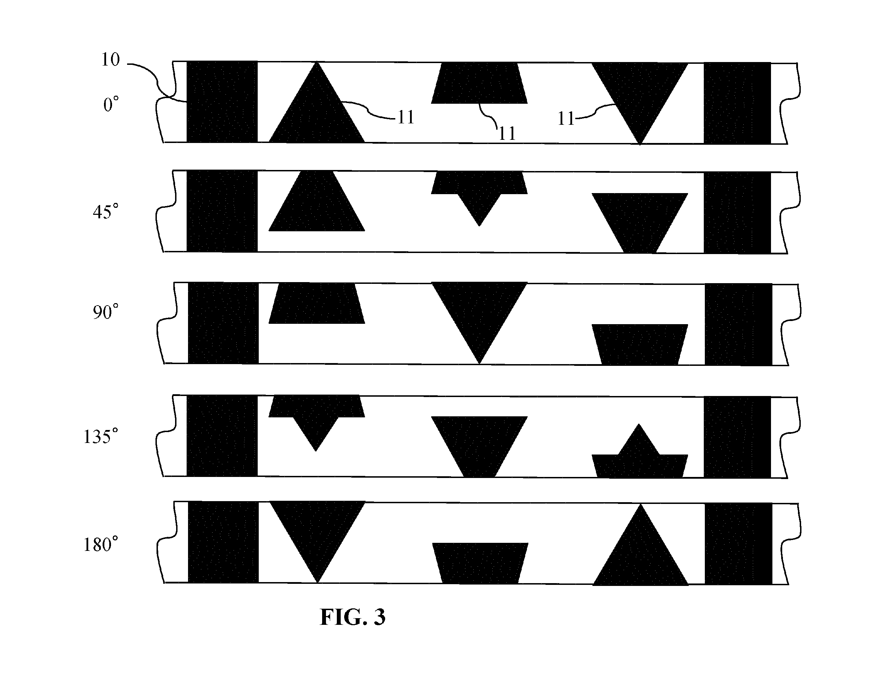

- Figur 3: zeigt eine zweidimensionale Darstellung (als "Draufsicht") eines erfindungs-gemäßen Ballonkatheters mit auf der Oberfläche angeordneten Markern in un-terschiedlichen Stadien der Rotation.

Nachfolgend wird die Erfindung anhand von Ausführungsbeispielen näher erläutert.

### Ausführungsbeispiel 1

In Figur 1A ist eine exzentrische Stenose schematisch dargestellt. Die Figur 1A zeigt einen Querschnitt durch ein Gefäß mit einer exzentrischen Stenose. Das Gefäßlumen 1 ist beeinträchtigt durch Ablagerungen 3, die allerdings asymmetrisch, nur an einer Seite der Gefäßwand auftreten. Umliegende gesunde Bereiche 2 der Gefäßwand weisen keine Ablagerungen 3 auf und benötigen daher keine Behandlung.

In Figur 1B ist eine erste Ausführungsform des erfindungsgemäßen Ballonkatheters dargestellt, wobei die Figur 1B einen Querschnitt durch den radial asymmetrisch dilatierbaren Abschnitt des Ballonkatheters zeigt. Dargestellt ist das Katheterlumen 4, welches für die Dilatation mit einem Fluid versehen werden kann. Das Katheterlumen 4 wird von einer Umfangswandung des dilatierbaren Ballons umgeben. Im radial asymmetrisch dilatierbaren Abschnitt weist diese Umfangswandung im Querschnitt einen ersten Bereich 6 und einen dem ersten Bereich 6 gegenüberliegenden, zweiten Bereich 5 auf. Der erste Bereich 6 und der zweite Bereich 5 unterscheiden sich voneinander in der Wandstärke und - dadurch bedingt - in der Dehnbarkeit der Umfangswandung. Die Wandstärke des zweiten Bereichs 5 ist geringer als die Wandstärke des ersten Bereichs 6. Dadurch dehnt sich der Ballon an dieser Stelle der Umfangswandung stärker als im ersten Bereich 6. Dies hat zur Folge, dass der resultierende Druck des Ballons im zweiten Bereich 5, der auf die Gefäßwand ausgeübt wird, größer ist als im ersten Bereich 6.

In Figur 1C ist schematisch dargestellt, wie der erfindungsgemäße Ballonkatheter im zu behandelnden Gefäß positioniert wird. Dabei zeigt der zweite Bereich 5 des Ballonkatheters zum Gefäßwandareal, welches die das Gefäßlumen 1 verengende Ablagerung 3 trägt.

Nach erfolgter Positionierung und Dilatation des dilatierbaren Ballons des erfindungsgemäßen Ballonkatheters wird der Zustand erreicht, der in Figur 1D schematisch dargestellt ist. Die Umfangswandung des dilatierbaren Ballons legt sich an die Gefäßwand an, wobei der durch die Umfangswandung im zweiten Bereich 5 auf die Gefäßwand ausgeübte resultierende Druck größer ist als im ersten Bereich 6. Dabei deutet 12 auf den Zustand vor der nominalen Druckbeaufschlagung hin und 13 beschreibt die Position des enddilatierten Ballonumfangs. Dadurch wird gezielt auf die Ablagerung 3 eine therapeutisch notwendige radial Kraft ausgeübt, während die umliegenden gesunden Teile der Gefäßwand weniger stark belastet werden und damit weniger anfällig sind für Verletzungen.

### Ausführungsbeispiel 2

In Figur 2 sind ausgewählte Gestaltungen von radial asymmetrisch dilatierbaren Abschnitten eines erfindungsgemäßen Ballonkatheters schematisch dargestellt. In Figur 2A ist ein radial asymmetrisch dilatierbarer Abschnitt 7 mit einem zweiten Bereich 9 in Streifenform gezeigt. Dabei erstreckt sich der zweite Bereich 9 über die gesamte Ausdehnung entlang der Längsachse des radial asymmetrisch dilatierbaren Abschnitts 7. Der zweite Bereich 9 wird seitlich von einem Bereich 8 begrenzt, der eine im Vergleich zum zweiten Bereich 9 größere Wandstärke aufweist.

In Figur 2B ist ein radial asymmetrisch dilatierbarer Abschnitt schematisch dargestellt, der einen zweiten Bereich in Ellipsenform aufweist.

In Figur 2C ist ein radial asymmetrisch dilatierbarer Abschnitt gezeigt, der zwei zweite Bereiche aufweist, wobei in diesem Fall beide zweiten Bereiche exemplarisch in ellipsenform gezeigt sind. Die zweiten Bereiche können natürlich auch andere Formen annehmen.

### Ausführungsbeispiel 3

In Figur 3 ist eine Anordnung von Markern auf der Oberfläche eines erfindungsgemäßen Ballonkatheters schematisch dargestellt, wobei die Marker derart positioniert und angeordnet sind, dass die Rotationslage des Ballonkatheters im Gefäß auch in einer zweidimensionalen Darstellung (als "Draufsicht") eindeutig bestimmbar ist. Der Ballonkatheter weist auf seiner Oberfläche Marker in Form von zwei Bändern 10 und mindestens einem Dreieck 11, bevorzugt zwei Dreiecken 11, besonders bevorzugt drei Dreiecken 11 auf.

Dabei sind die drei Dreiecke 11 derart orientiert und aufeinander abgestimmt, dass sich aus deren zweidimensionalem Bild exakt und eindeutig die Rotationslage des Ballonkatheters im Gefäß bestimmen lässt. Dazu sind die drei Dreiecke 11 in ihrer Ausrichtung der Spitze alternierend angeordnet. Die Dreiecke 11 erstrecken sich in ihrer Ausdehnung von der Spitze bis zur Basis im Wesentlichen jeweils über den halben Umfang des Ballonkatheters. Wie in Figur 3 gezeigt ergibt sich aus dieser Anordnung der Dreiecke 11 für jede Rotationslage (0° bis 180°) ein eindeutiges zweidimensionales Bild, so dass die Bestimmung der Rotationslage anhand von erhaltenen zweidimensionalen Bildern bestimmt werden kann. Dabei ist es ausreichend, dass mindestens ein Dreieck 11 abgebildet wird. Um allerdings den Effekt von Artefaktbildung während der Bildgebung zu minimieren, kann es sinnvoll sein, mehrere dieser Dreiecke 11 in unterschiedlicher Rotation und Orientierung zueinander zu verwenden, wobei zwei Dreiecke die selbe Orientierung aber unterschiedliche Rotationslagen aufweisen können. Bevorzugt werden Röntgenmarker, Fluoreszenzmarker und/oder Kombinationen daraus eingesetzt.

## Patentansprüche

1. Ballonkatheter mit einem dilatierbaren Ballon, **dadurch gekennzeichnet, dass** der dilatierbare Ballon mindestens einen radial asymmetrisch dilatierbaren Abschnitt umfasst, wobei der Querschnitt des radial asymmetrisch dilatierbaren Abschnitts mindestens einen ersten Bereich und einen, dem ersten Bereich gegenüberliegenden, zweiten Bereich aufweist und sich der erste und der zweite Bereich in ihrer Radialfestigkeit der Ballonwand voneinander unterscheiden.

2. Ballonkatheter nach Anspruch 1, **dadurch gekennzeichnet, dass** an der der minimalen Wandstärke des zweiten Bereichs gegenüberliegenden Stelle des Querschnittsumfangs die Wandstärke des ersten Bereichs größer ist als die minimale Wandstärke des zweiten Bereichs.

3. Ballonkatheter nach einem der vorhergehenden Ansprüche, **dadurch gekennzeichnet, dass** der zweite Bereich nicht mehr als 50% und nicht weniger als 0,01% des Querschnittsumfangs umfasst.

4. Ballonkatheter nach einem der vorhergehenden Ansprüche, **dadurch gekennzeichnet, dass** der Ballonkatheter mehr als einen radial asymmetrisch dilatierbaren Abschnitt aufweist.

5. Ballonkatheter nach einem der vorhergehenden Ansprüche, **dadurch gekennzeichnet, dass** der Ballonkatheter einen radial asymmetrisch dilatierbaren Abschnitt mit mehr als einem zweiten Bereich aufweist.

6. Ballonkatheter nach einem der vorhergehenden Ansprüche, **dadurch gekennzeichnet, dass** der radial asymmetrisch dilatierbare Abschnitt sich im Wesentlichen über den gesamten dilatierbaren Ballon erstreckt.

7. Ballonkatheter nach einem der Ansprüche 1 bis 5, **dadurch gekennzeichnet, dass** die Summe der Längsausdehnung der radial asymmetrisch dilatierbaren Abschnitte nicht mehr als 50% der Längsausdehnung des dilatierbaren Bereichs des Ballonkatheters beträgt.

8. Ballonkatheter nach einem der vorhergehenden Ansprüche, **dadurch gekennzeichnet, dass** das Verhältnis δ der minimalen Wandstärke des zweiten Bereichs zur Wandstärke der der minimalen Wandstärke des zweiten Bereichs gegenüberliegenden Stelle des ersten Bereichs 1>δ≥0,01 beträgt, bevorzugt 0,95≥δ≥0,1.

9. Ballonkatheter nach einem der vorhergehenden Ansprüche, **dadurch gekennzeichnet, dass** die Oberfläche des dilatierbaren Ballons einen oder mehrere Marker aufweist, die derart angebracht und orientiert sind, dass während der Positionierung des Ballonkatheters in einem Gefäß des zu behandelnden Individuums die räumliche Ausrichtung des radial asymmetrisch dilatierbaren Abschnitts bestimmbar ist, bevorzugt ist die Rotationsstellung des radial asymmetrisch dilatierbaren Abschnitts bestimmbar.

10. Ballonkatheter nach Anspruch 9, **dadurch gekennzeichnet, dass** es sich bei den Markern um Substanzen handelt, die durch bildgebende Verfahren detektierbar und/oder darstellbar sind, bevorzugt handelt es sich um Röntgen- und/oder Fluoreszenzmarker.

11. Ballonkatheter nach einem der Ansprüche 9 oder 10, **dadurch gekennzeichnet, dass** mindestens zwei verschiedene Marker verwendet werden, bevorzugt mindestens drei verschiedene Marker.

12. Ballonkatheter nach einem der Ansprüche 9 bis 11, **dadurch gekennzeichnet, dass** die Marker in unterschiedlichen oder gleichen geometrischen Figuren auf der Oberfläche angeordnet sind.

13. Ballonkatheter nach Anspruch 12, **dadurch gekennzeichnet, dass** die geometrischen Figuren Ringe, Bänder, Dreiecke und/oder Pfeile umfassen oder daraus bestehen.

14. Ballonkatheter nach Anspruch 12 oder 13, **dadurch gekennzeichnet, dass** mindestens drei gleiche oder verschiedene geometrische Figuren derart auf der Oberfläche des dilatierbaren Ballons ausgerichtet sind, so dass die Rotationsstellung des oder der radial asymmetrisch dilatierbaren Abschnitte bestimmbar ist.
